# EUROPEAN PATENT APPLICATION

(11) **EP 4 389 875 A1**
(43) Date of publication of application: **26.06.2024**
(21) Application number: 21953875.8
(22) Date of filing: 31.08.2021
(51) Int. Cl.: C12N 5/00, C12N 5/0781

(54) **CULTURE MEDIUM AND CULTURE METHOD FOR HUMAN PRIMARY ACUTE MYELOID LEUKEMIA CELLS**

(30) Priority: 20.08.2021 CN 202110959221
(71) Applicant: Precedo Pharmaceuticals Co., Ltd., Hefei, Anhui 230094 (CN)
(72) Inventor: LIU, Qing Song, Hefei, Anhui 230031 (CN); HE, Yu Ying, Hefei, Anhui 230031 (CN); CHEN, Cheng, Hefei, Anhui 230031 (CN); HUANG, Tao, Hefei, Anhui 230031 (CN); REN, Tao, Hefei, Anhui 230031 (CN); WANG, Wen Chao, Hefei, Anhui 230031 (CN); WANG, Li, Hefei, Anhui 230031 (CN)
(74) Representative: Abel & Imray LLP
(86) International application number: PCT/CN2021/115503
(87) International publication number: WO 2023/019638

(57) **Abstract**

Provided are a culture medium and a culture method for human primary acute myeloid leukemia cells. The culture medium for human primary acute myeloid leukemia cells contains a glutamine additive, non-essential amino acids, human interleukin-6, human interleukin-7, human interleukin-3, recombinant human FLT3 Ligand, a recombinant human macrophage colony stimulating factor and a human stem cell factor. Acute myeloid leukemia cells can be cultured with higher amplification efficiency and longer in-vitro culture time by using the culture medium and culture method. Also provided are human primary acute myeloid leukemia cells cultured in vitro by using the culture medium, and the use thereof for curative effect evaluation and screening of drugs.

## Description

### Technical field

The invention relates to the field of cell culture technology, in particular to a primary cell culture medium for culturing human primary acute myeloid leukemia (AML) cells *in vitro,* a method for using the culture medium to culture human primary acute myeloid leukemia cells, and application thereof in efficacy evaluating and screening of drugs.

### Background of the Invention

Acute myeloid leukemia (AML) is a clonal malignant proliferative blood disease of myeloid blasts in the hematopoietic system. AML is an aggressive and highly allogenic disease with biologically and prognostically distinct subtypes. AML infects 1 to 5 people per 100,000 people each year and accounts for 30%-40% of all new leukemia cases. AML is the most lethal leukocyte disease with the worst prognosis and survival (<26% at 5 years), which has not improved since the 1970s (Hanyang Lin, et al., Feeder-free and serum-free in vitro assay for measuring the effect of drugs on acute and chronic myeloid leukemia stem/progenitor cells, Experimental Hematology 2020; 90: 52-64).

To date, AML stem cells have been the main focus of leukemia research because they have acquired resistance and mediate relapsed leukemia subclones. The research work typically requires culturing AML stem cells. Culture of AML stem cells is challenging due to the heterogeneity within the population of AML stem cells and the fact that many patients have poorly grown cells that are prone to differentiation and lose their ability to self-renew *in vitro.* Additionally, it might be difficult to obtain primary AML patient samples, and unlike transformed cell lines, they are not able to indefinitely expand *in vitro.* In vivo xenograft models allow expansion of human AML cells but require large numbers of cells (>10⁶ cells/mouse) and do not support maintenance of AML cells. On the other hand, co-culture with bone marrow (BM)-derived mesenchymal stromal cells (MSCs) or stromal cell lines can help preserve AML stem cells, but cultures of feeder cells are not suitable for high-throughput drug screening or adaptive testing.

Therefore, there is a need in this field for a culture medium and culture method for long-term, rapid expansion of human primary acute myeloid leukemia cells without co-culture with stromal cells.

### Summary of the Invention

In order to solve the above technical problems, the invention provides a culture medium and a culture method for rapid expansion of human primary acute myeloid leukemia cells *in vitro.*

One aspect of the invention is to provide a culture medium for human primary acute myeloid leukemia cells, the culture medium comprising a glutamine additive, non-essential amino acid(s), human interleukin-6 (human IL-6), human interleukin-7 (human IL-7), human interleukin-3 (human IL-3), recombinant human FLT3 Ligand (human FLT3L), recombinant human macrophage colony-stimulating factor (human M-CSF), and human stem cell factor (human SCF).

In a preferred aspect of the invention, the culture medium for human primary acute myeloid leukemia cells satisfies any one, more or all of the following conditions:
(1) the amount of the glutamine additive in the culture medium is preferably 0.5 mM to 4 mM;
(2) the non-essential amino acid(s) is/are one or more selected from the group consisting of glycine, alanine, asparagine, aspartic acid, glutamic acid, proline and serine, and the amount of the non-essential amino acid(s) in the culture medium is preferably 12.5 µM to 200 µM;
(3) the amount of human IL-6 in the culture medium is preferably 1.89 ng/mL to 17 ng/mL;
(4) the amount of human IL-7 in the culture medium is preferably 1.89 ng/mL to 51 ng/mL;
(5) the amount of human IL-3 in the culture medium is preferably 1.89 ng/mL to 153 ng/mL;
(6) the amount of the human FLT3L in the culture medium is preferably 3 ng/mL to 81 ng/mL;
(7) the amount of the human M-CSF in the culture medium is preferably 1 ng/mL to 81 ng/mL;
(8) the amount of the human SCF in the culture medium is preferably 1 ng/mL to 81 ng/mL.

In another preferred embodiment, the culture medium for human primary acute myeloid leukemia cells of the invention further comprises a Basal Medium which comprises an initial medium selected from the group consisting of monocyte serum-free medium and RPMI-1640, 5-10% (v/v) fetal bovine serum, and one or more antibiotic(s) selected from the group consisting of streptomycin/penicillin, amphotericin B, and Primocin. Specifically, in case of using streptomycin/penicillin, the concentration range of streptomycin is 25-400 µg/mL, preferably 50-200 µg/mL, the concentration range of penicillin is 25-400 U/mL, preferably 50-200 U/mL; in case of using amphotericin B, the concentration range is 0.25-4 µg/mL, preferably 0.5-2 µg/mL; and in case of using Primocin, the concentration range is 25-400 µg/mL, preferably 50-200 µg/mL.

On the other hand, the invention also provides a method for culturing human primary acute myeloid leukemia cells *in vitro,* including a step of culturing human primary acute myeloid leukemia cells *in vitro* using the culture medium for human primary acute myeloid leukemia cells of the invention.

In a preferred embodiment, the method for culturing human primary acute myeloid leukemia cells *in vitro* of the invention comprises the following steps:
1. Isolating and processing human primary acute myeloid leukemia cells
   (1) Centrifuging the bone marrow samples from patients with acute myeloid leukemia at 1200-1600 rpm for 2 to 6 minutes.
   (2) After centrifugation, discarding the upper blood plasma layer, and adding 1 x PBS in amount of 2 to 3 times the amount of the blood cell precipitation to the blood cell precipitation for dilution, and mixing them thoroughly; adding 6-8 mL Human Peripheral Blood Lymphocyte Separation Medium to the above diluted blood cell precipitation and centrifuging the resultant at a speed of 380-420 g, acceleration of 1-2, deceleration of 0, temperature of 20-28°C, and centrifugation time of 25-35 minutes.
   (3) After centrifugation to form layers in the centrifuge tube, aspirating the lymphocyte layer into 3-6 mL 1x PBS, and mixing them well to wash the cells and then centrifuging them at a speed of 1200-1600 rpm for 2-6 minutes.
   (4) Discarding the supernatant, adding Red Blood Cell Lysis Buffer to resuspend the cell pellet, and lysing for 15 to 20 minutes, centrifuging at 1200 to 1600 rpm for 2 to 6 minutes.
   (5) Discarding the supernatant after centrifugation, and adding Basal Medium for later use.
2. Culturing the cells using the culture medium for human primary acute myeloid leukemia cells of the invention

Resuspending the human primary acute myeloid leukemia cells obtained in the above step 1 in the culture medium for human primary acute myeloid leukemia cells of the invention and counting the cells, inoculating the cells in a culture dish at a density of 1 × 10⁵ to 4 × 10⁶ cells/cm², and passaging the cells when the cells grow to cover 90% of the culture dish.

In yet another aspect, the cells obtained by the culture method of the invention may be used in regenerative medicine, basic medical research on acute myeloid leukemia cells, screening of drug response, and development of new drugs for acute myeloid leukemia. Therefore, the invention also provides a method for screening or efficacy evaluating of drugs for human primary acute myeloid leukemia, comprising the steps of:
(1) culturing the human primary acute myeloid leukemia cells by the method for culturing human primary acute myeloid leukemia cells of the invention;
(2) selecting the drug to be tested and diluting the drug into different concentration gradients;
(3) adding the drug which has been diluted to the cells cultured and obtained in step(1), and detecting the cell viability.

The technical solution of the invention can achieve the following technical effects:
(1) the success rate for culturing the human primary acute myeloid leukemia cells is improved, with a success rate of 80% or more;
(2) the human primary acute myeloid leukemia cells cultured *in vitro* is ensured to maintain the pathological characteristics of patients;
(3) the human primary acute myeloid leukemia cells are expanded with high efficiency, with a magnitude of 10⁶ of human primary acute myeloid leukemia cells being successfully expanded within about one week from the starting of 10⁵-level cell count, and the expanded human primary acute myeloid leukemia cells have the ability of continuous passage;
(5) the cost for culture is controllable, as the culture medium does not require expensive factors such as Wnt agonists, R-spondin family proteins, BMP inhibitors, FGF10;
(6) this technology can culture and provide the human primary acute myeloid leukemia cells with large quantity and high uniformity, which is suitable for high-throughput screening of new candidate compounds and high-throughput drug sensitivity functional tests *in vitro* for patients.

### Description of Drawings

Figure 1 is a graph showing the effects of different combinations of added factors on the proliferation of human primary acute myeloid leukemia cells.
Figures 2A-2H are graphs showing the effects of different concentrations of each added factor on the proliferation of human primary acute myeloid leukemia cells.
Figure 3 is a photograph taken under a microscope of human primary acute myeloid leukemia cells cultured using the culture medium for human primary acute myeloid leukemia cells of the invention.
Figure 4 is a image showing the results of flow cytometric identification of human primary acute myeloid leukemia cells cultured using the culture medium for human primary acute myeloid leukemia cells of the invention.
Figure 5 is a cell growth curve of human primary acute myeloid leukemia cells cultured *in vitro* using the culture medium for human primary acute myeloid leukemia cells of the invention.
Figure 6 is a comparison graph of culturing human primary acute myeloid leukemia cells using the culture medium for human primary acute myeloid leukemia cells of the invention and the prior art culture medium.
Figures 7A-7F are dose-response curves of different passages of human primary acute myeloid leukemia cells cultured using the culture medium for human primary acute myeloid leukemia cells of the invention for different drugs.

### Detailed Description of the Invention

In order to better understand the invention, it is further described below in combination with the examples and the drawings. The following examples are only for the purpose of illustrating the invention, but not for the purpose of defining.

### Example 1. Effects of respective added factors in the culture medium for human primary acute myeloid leukemia cells on the proliferation of human primary acute myeloid leukemia cells

### (1) Preparation of culture mediums for human primary acute myeloid leukemia cells

First, the Basal Medium was prepared. The formulation of the Basal Medium was monocyte serum-free medium (purchased from BI, 05-080-1A) + 10% (v/v) fetal bovine serum (purchased from ExCell Bio, FND500) + 100 µg/ mL Primocin (purchased from InvivoGen, 0.2% (v/v), the commercial product concentration is 50 mg/mL).

Different types of growth factors (see Table 1) were added to the Basal Medium to prepare culture mediums for human primary acute myeloid leukemia cells containing different added components.

### (2) Isolation and processing of human primary acute myeloid leukemia cells

### 1 Sample selection

Bone marrow samples were obtained from AML patients by professional medical staff in a professional medical institution, and all patients have signed informed consent forms. Bone marrow samples of 3 to 10 mL were stored in EDTA-K2 anticoagulant tubes (manufacturer: Jiangsu Rongye) and transported refrigerated at 4 to 8°C.

### 2 Material preparation

After surface sterilization, 15 mL sterile centrifuge tubes, pipettors, 10 mL pipettes, and sterile pipette tips were placed into a ultra clean workbench and exposed to ultraviolet irradiation for 30 minutes. 1 × PBS was taken out from the 4°C refrigerator 30 minutes in advance.

### 3 Sample isolation

3.1 In a ultra clean workbench, the bone marrow samples were mixed by pipetting up and down, and transferred to a 15 mL centrifuge tube, and centrifuged at 1500 rpm for 4 minutes at room temperature;
3.2 6 mL of Human Peripheral Blood Lymphocyte Separation Medium (purchased from Solarbio, P8610) was added to a new 15 mL centrifuge tube; after centrifugation of the bone marrow sample, the upper blood plasma layer was discarded, and 1 × PBS in amount of 2 to 3 times the amount of the blood cell precipitation was added to the blood cell precipitation for dilution, and mixed thoroughly; the diluted blood was slowly superimposed on the surface of the separation medium layer along the wall of the centrifuge tube, paying attention to keeping the liquid surface clear; the centrifuge tube of the bone marrow sample mixture was gently placed into a centrifuge, and centrifuged at 400 g for 30 minutes (acceleration: 2, deceleration: 0, temperature: 25°C);
3.3 after centrifugation, the cells in the centrifuge tube were separated into four layers from top to bottom (PBS layer, ring-shaped milky white lymphocyte layer, separation medium layer, and red blood cell layer); the lymphocyte layer was pipetted in a circle manner into a 15 mL centrifuge tube which has been pre-added with 5 mL of 1 × PBS; the resultant was mixed gently to wash the cells, and centrifuged at 1500 rpm for 5 minutes at room temperature;
3.4 the supernatant was discarded, and the resultant was observed to determine whether there were blood cells; if there were blood cells, 8 mL of Red Blood Cell Lysing Buffer (purchased from Sigma, R7757-100ML) was added, which was then mixed well, lysed at 4°C for 20 minutes, with reverse mixing once during the process; the resultant was centrifuged at 1500 rpm at room temperature for 4 minutes;
3.5 the supernatant was discarded and the resultant was added with 2 mL of Basal Medium to resuspend the cells for later use.

### 4 Counting and processing of cells

4.1 Viable cell counting: 12 µL of the resuspended cell suspension was fully mixed with 12 µL of trypan blue dye (Sangon Biotech (Shanghai) Co., Ltd.), and then 20 µL of the mixture was added into a cell counting plate (Countstar, specifications: 50 pieces/box); the percentage of viable large cells (cell size >10 µm) were calculated using a cell counter (Countstar, IC1000) (percentage of viable large cells (cell size >10 µm)= number of viable cells / total number of cells*100%).

### (3) Culture of human primary acute myeloid leukemia cells

The culture mediums of different compositions in Table 1 were added into a 96-well plate in a volume of 100 µL/well. Human primary acute myeloid leukemia cells isolated from two cases of human primary acute myeloid leukemia bone marrow samples (numbered A17007, A25104, from the First Affiliated Hospital of Anhui Medical University) according to the above step (2) were inoculated in the 96-well culture plate at a density of 1 × 10⁴ cells/well and cultured at 37°C and 5% CO₂. After 5 to 8 days of culture, the cells grew to 70% to 85%, and 10 µL of Cell Counting Kit-8 (CCK-8, purchased from MCE) was added to each well and incubated at 37°C and 5% CO₂ for 2 to 4 hours. The substances in each well were mixed, and the plate was read using a multifunctional microplate reader (Multi-Mode Detection Platform, American Molecular Instruments (Shanghai) Co., Ltd.) at 450 nm. The Basal Medium without addition of any additive was used as a control. The experimental results are shown in Table 1.

**Table 1 Added components in the culture medium and their effects on promoting cell proliferation**

| No. | Name | Abbreviation | Supplier | Final concentration | Level of promoting cell proliferation |
|---|---|---|---|---|---|
| 1 | human interleukin-2 | human IL-2 | Sino Biological | 1 ng/mL | - |
| 2 | human granulocyte colony stimulating factor | human G-CSF | Sino Biological | 17ng/mL | - |
| 3 | human interleukin-7 | human IL-7 | Sino Biological | 1.89 ng/mL | + |
| 4 | human leukocyte interferon | human IFN-α | Sino Biological | 51ng/mL | + |
| 5 | tumor necrosis factor-a | human TNF-α | Sino Biological | 51ng/mL | ∘ |
| 6 | recombinant human macrophage colony-stimulating factor | human M-CSF | Sino Biological | 27ng/mL | + |
| 7 | human interleukin-5 | human IL-5 | Sino Biological | 17ng/mL | - |
| 8 | human interleukin-22 | human IL-22 | Peprotech | 51ng/mL | - |
| 9 | human thrombopoietin | human TPO | Sino Biological | 3ng/mL | - |
| 10 | human stem cell factor | human SCF | Sino Biological | 20ng/mL | + |
| 11 | human interleukin-6 | human IL-6 | Sino Biological | 51ng/mL | + |
| 12 | human interleukin-4 | human IL-4 | Sino Biological | 51ng/mL | - |
| 13 | glutamine additive | - | GIBCO | 1mM | + |
| 14 | recombinant human FLT3 Ligand | human FLT3L | Sino Biological | 3 ng/mL | + |
| 15 | human interleukin-3 | human IL-3 | Sino Biological | 51ng/mL | + |
| 16 | Y-27632 | Y-27632 | MCE | 10µM | - |
| 17 | human interleukin-10 | human IL-10 | Sino Biological | 17ng/mL | - |
| 18 | human interleukin-11 | human IL-11 | Sino Biological | 0.63ng/mL | - |
| 19 | non-essential amino acid(s) | - | Corning | 50µM | + |

| | | | | | |
|---|---|---|---|---|---|
| Wherein, "+" indicates that compared with the Basal Medium, the culture medium added with the additive can promote the proliferation of two cases of human primary acute myeloid leukemia cells isolated from human primary acute myeloid leukemia bone marrow samples; "-" indicates that the culture medium added with the additive can promote the proliferation of at least one case of human primary acute myeloid leukemia cells isolated from human primary acute myeloid leukemia bone marrow samples; "o" indicates that the medium added with the additive has no significant effect on the proliferation of at least two cases of human primary acute myeloid leukemia cells isolated from human primary acute myeloid leukemia bone marrow samples. | | | | | |

Based on the above results, factors such as human IL-7, human IFN-α, human M-CSF, human SCF, human IL-6, glutamine additives, human FLT3L, human IL-3, and non-essential amino acids were selected for further culture experiments in Example 2.

### Example 2. Effects of different combinations of added factors in the culture medium for human primary acute myeloid leukemia cells on the proliferation of human primary acute myeloid leukemia cells

The culture mediums for human primary acute myeloid leukemia cells containing different combinations of added factors were prepared according to the components in Table 2, to investigate the proliferation-promoting effects of different added factor combinations on human primary acute myeloid leukemia cells.

**Table 2 Preparation of culture mediums with different components (final concentrations are shown)**

| Culture medium | | Composition | |
|---|---|---|---|
| Basal (BM) Medium | | monocyte serum-free medium + 10 %(v/v) fetal bovine serum + 100 µg/mL Primocin | |
| | No.1 | BM + 51 ng/ml human IFN-α + 1mM glutamine additive+ 20 ng/ml human SCF + 51 ng/ml human IL-6 + 51 ng/ml human IL-3 + 3 ng/ml human FLT3L + 50 µM non-essential amino acids + 1.89 ng/ml human IL-7 + 27 ng/ml M-CSF | |
| | No.2 | | No.1 - 51 ng/ml human IFN-α |
| | No.3 | | No.1 - 1mM non-essential amino acids |
| | No.4 | | No.1 - 20 ng/ml human SCF |
| | No.5 | | No.1 - 51 ng/ml human IL-6 |
| | No.6 | | No.1 - 51 ng/ml human IL-3 |
| | No.7 | | No.1 - 3 ng/ml human FLT3L |
| | No.8 | | No.1 - 50 µM non-essential amino acids |
| | No.9 | | No.1 - 1.89 ng/ml human IL-7 |
| | No.10 | | No.1 - 27 ng/ml M-CSF |

Human primary acute myeloid leukemia cells were obtained from human primary acute myeloid leukemia bone marrow samples (numbered A17151, A17152, A20090, A20101, A21004, A20141) according to the process of step (2)-3 of Example 1. The obtained cell suspension was divided into 11 equal parts, which were then centrifuged at 1500 rpm for 4 minutes. After centrifugation, the cells were resuspended with 200 µL BM or No. 1 to 10 culture medium, respectively, and inoculated into a 48-well plate at a viable cell density of 2 × 10⁴ cells/well (20,000 cells per well). Each well in the 48-well plate was supplemented to a volume of 1 mL with the corresponding culture mediums, and the resultant was fully mixed. After surface disinfection, the plate was placed in a 37°C, 5% CO₂ incubator (purchased from Thermo Fisher) for culture.

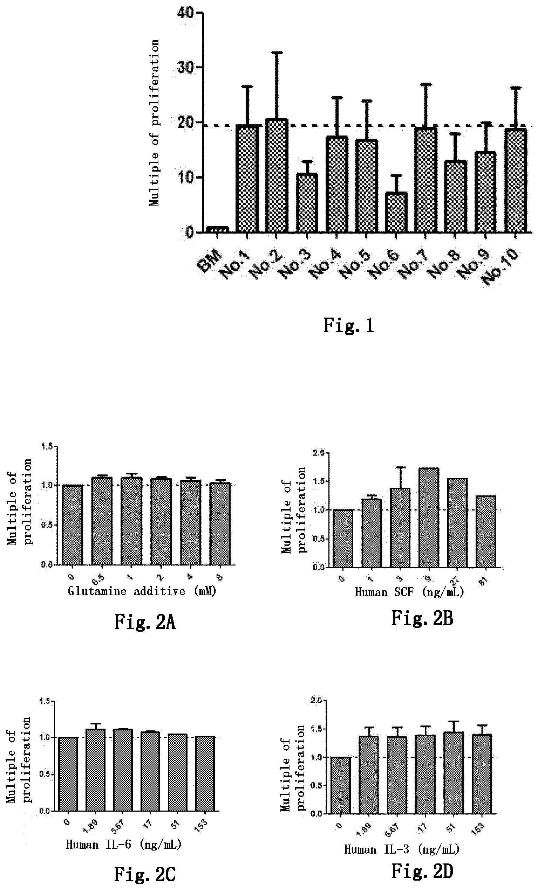

The cells in the 48-well plate grew to more than 85% and were transferred to a 15 mL centrifuge tube which was then centrifuged at 1500 rpm for 5 minutes. 500 µL of monocyte serum-free medium was added to the centrifuge tube to resuspend the cell pellet. 12 µL of the resuspended cell suspension was fully mixed with 12 µL of trypan blue dye (Sangon Biotech (Shanghai) Co., Ltd.), and then 20 µL of the mixture was added into a cell counting plate (Countstar, specifications: 50 pieces/box). The percentage of viable large cells (cell size >10 µm) were calculated using a cell counter (Countstar, IC1000) (percentage of viable large cells (cell size >10 µm) = number of viable cells / total number of cells*100%). The results obtained from human primary acute myeloid leukemia cells of bone marrow samples A17151, A17152, A20090, A20101, A21004, and A20141 are shown in Figure 1.

According to the results in Figure 1, it can be seen that compared with the Basal Medium, the use of the No.1 - No.10 culture mediums can promote the proliferation of human primary acute myeloid leukemia cells to varying degrees. The formulation of No. 2 culture medium does not comprise human IFN-α, which surprisingly leads to a better proliferation effect. The results show that factors such as glutamine additive, human SCF, human IL-6, human IL-3, human FLT3L, non-essential amino acids, human IL-7 and human M-CSF may significantly promote the proliferation of human primary acute myeloid leukemia cells

### Example 3. Proliferative effects of different concentrations of added factors on human primary acute myeloid leukemia cells

Human primary acute myeloid leukemia cells were obtained from human primary acute myeloid leukemia bone marrow samples (numbered A23065, A17112) according to the process of step (2)-3 of Example 1. The cells were resuspended with Basal Medium (monocyte serum-free medium + 10% (v/v) fetal bovine serum + 100 µg/mL Primocin) for later use.

Next, the factors with cell culture proliferation-promoting effect determined in Example 2 were added to the Basal Medium (prepared according to the No. 2 formulation of Example 2) to obtain a combined basal medium, and then the following 8 formulations of culture mediums were prepared for experiments. :
Formulation 1: the combined basal medium without glutamine additive;
Formulation 2: the combined basal medium without human SCF;
Formulation 3: the combined basal medium without human IL-6;
Formulation 4: the combined basal medium without human IL-3;
Formulation 5: the combined basal medium without human FLT3L;
Formulation 6: the combined basal medium without non-essential amino acids;
Formulation 7: the combined basal medium without human IL-7;
Formulation 8: the combined basal medium without human M-CSF.

20 µl of cell suspension containing 4 × 10⁴ cells was added to each well, and the suspension was diluted with 1mL medium of the above Formulations 1-8, respectively.

When using the culture medium of Formulation 1, the prepared glutamine additive was added respectively to a 48-well plate inoculated with primary cells, with 1 mL per well, and the final concentration of glutamine additive was 0.5 mM, 1 mM, 2 mM, 4 mM, 8 mM, respectively; a well of Blank Control (BC) was set using the culture medium of Formulation 1.

When using the culture medium of Formulation 2, the prepared human SCF was added respectively to a 48-well plate inoculated with primary cells, with 1 mL per well, and the final concentration of human SCF was 1 ng/mL, 3 ng/mL, 9 ng/mL, 27 ng/mL, 81 ng/mL, respectively; a well of Blank Control (BC) was set using the culture medium of Formulation 2.

When using the culture medium of Formulation 3, the prepared human IL-6 was added respectively to a 48-well plate inoculated with primary cells, with 1 mL per well, and the final concentration of human IL-6 was 1.89 ng/mL, 5.67 ng/mL, 17 ng/mL, 51 ng/mL, 153 ng/mL, respectively; a well of Blank Control (BC) was set using the culture medium of Formulation 3.

When using the culture medium of Formulation 4, the prepared human IL-3 was added respectively to a 48-well plate inoculated with primary cells, with 1 mL per well, and the final concentration of human IL-3 was 1.89 ng/mL, 5.67 ng/mL, 17 ng/mL, 51 ng/mL, 153 ng/mL, respectively; a well of Blank Control (BC) was set using the culture medium of Formulation 4.

When using the culture medium of Formulation 5, the prepared human FLT3L was added respectively to a 48-well plate inoculated with primary cells, with 1 mL per well, and the final concentration of human FLT3L was 1 ng/mL, 3 ng/mL, 9 ng/mL, 27 ng/mL, 81 ng/mL, respectively; a well of Blank Control (BC) was set using the culture medium of Formulation 5.

When using the culture medium of Formulation 6, the prepared non-essential amino acids were added respectively to a 48-well plate inoculated with primary cells, with 1 mL per well, and the final concentration of non-essential amino acids was 12.5 µM, 25 µM, 50 µM, 100 µM, 200 µM, respectively; a well of Blank Control (BC) was set using the culture medium of Formulation 6.

When using the culture medium of Formulation 7, the prepared IL-7 was added respectively to a 48-well plate inoculated with primary cells, with 1 mL per well, and the final concentration of IL-7 was 1.89 ng/mL, 5.67 ng/mL, 17 ng/mL, 51 ng/mL, 153 ng/mL, respectively; a well of Blank Control (BC) was set using the culture medium of Formulation 7.

When using the culture medium of Formulation 8, the prepared human M-CSF was added respectively to a 48-well plate inoculated with primary cells, with 1 mL per well, and the final concentration of human M-CSF was 1 ng/mL, 3 ng/mL, 9 ng/mL, 27 ng/mL, 81 ng/mL, respectively; a well of Blank Control (BC) was set using the culture medium of Formulation 8.

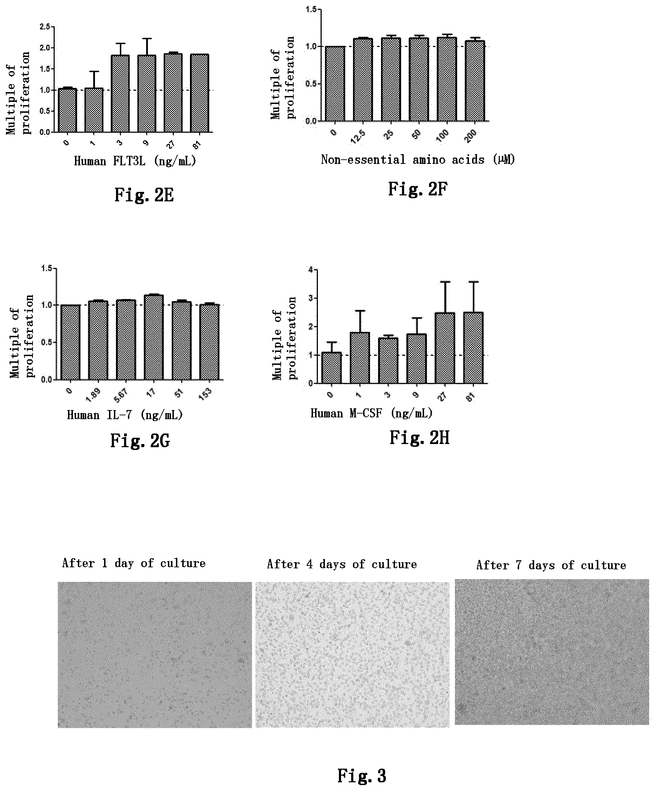

When the cells were expanded to about 85% of the 48 wells, the proliferation multiple were calculated by referring to the number of cells in the well of Blank Control (BC), and the results were shown in Figures 2A to 2H, respectively. In Figures 2A to 2H, the ratio is a ratio of the number of cells of the first passage cultured by using each culture medium to the number of cells of the first passage cultured in the corresponding well of Blank Control. If the ratio is greater than 1, it indicates that the proliferation promoting effect of the prepared culture medium containing different concentrations of factors or small molecular compounds is preferable over that of the culture medium in the well of Blank Control; if the ratio is less than 1, it indicates that the proliferation promoting effect of the prepared culture medium containing different concentrations of factors or small molecular compounds is poorer than that of the culture medium in the well of Blank Control.

According to the results in Figures 2A to 2H, glutamine additive, human SCF, human IL-6, human IL-3, human FLT3L, non-essential amino acids, human IL-7, and human M-CSF have a significant proliferation-promoting effect on primary human acute myeloid leukemia cells. According to the results of this example, the amount of glutamine additive is preferably 0.5 mM to 4 mM, and the cell proliferation effect is most significant when adding in a concentration of 0.5 mM; the amount of human SCF is preferably 1 ng/ml to 81 ng/ml, and the cell proliferation effect is most significant when adding in a concentration of 9 ng/ml; the amount of human IL-6 is preferably 1.89 ng/ml to 17 ng/ml, and the cell proliferation effect is most significant when adding in a concentration of 5.67 ng/mL; the amount of human IL-3 is preferably 1.89 ng/ml to 153 ng/ml, and the cell proliferation effect is most significant when adding in a concentration of 51 ng/mL; the amount of human FLT3L is preferably 3 ng/ml to 81 ng/ml, and the cell proliferation effect is most significant when adding in a concentration of 27 ng/mL; the amount of non-essential amino acids is preferably 12.5 µM to 200 µM, and the cell proliferation effect is most significant when adding in a concentration of 50 µM; the amount of human IL-7 is preferably 1.89 ng/ml to 51 ng/ml, and the cell proliferation effect is most significant when adding in a concentration of 17 ng/mL; the amount of human M-CSF is preferably 1 ng/ml to 81 ng/ml, and the cell proliferation effect is most significant when adding in a concentration of 27 ng/mL.

### Example 4. Culture and identification of human primary acute myeloid leukemia cells

### (1) Culture of human primary acute myeloid leukemia cells

Human primary acute myeloid leukemia cells were obtained from human primary acute myeloid leukemia bone marrow samples (numbered A17030) according to the process of step (2)-3 of Example 1, and cultured using the culture medium for human primary acute myeloid leukemia cells of the invention (the composition is the optimal components and concentration combination determined in Example 3, that is, comprising Basal Medium, 0.5 mM glutamine additive, 9 ng/mL human SCF, 5.67 ng/mL human IL-6, 51 ng/mL mL human IL-3, 27 ng/mL human FLT3L, 50 µM non-essential amino acids, 17 ng/mL human IL-7, and 27 ng/mL human M-CSF). The obtained human primary acute myeloid leukemia cells were inoculated into a 6-well plate at a viable cell density of 3 × 10⁶ cells/well. 5 mL of the culture medium for human primary acute myeloid leukemia cells of the invention was added into the plate and the resultant was mixed well. After surface disinfection, the plate was placed in a 37°C, 5% CO₂ incubator (purchased from Thermo Fisher) for culture.

The cultured human primary acute myeloid leukemia cells was observed under the microscope (EVOS M500, Invitrogen). Figure 3 shows the photos of 1 day, 4 days, and 7 days of culture taken under a 10x objective lens. According to cell counting, the number of viable cells increased to 3.58 times after 7 days of culture.

### (2) Flow cytometry identification of human primary acute myeloid leukemia cells

Human primary acute myeloid leukemia cells were obtained from human primary acute myeloid leukemia bone marrow samples (numbered A17014) according to the process of step (2)-3 of Example 1, and cultured using the culture medium for human primary acute myeloid leukemia cells of the invention. Specifically, the obtained human primary acute myeloid leukemia cells were inoculated in a 12-well plate at a viable cell density of 1 × 10⁶ cells/well. 3 mL of the culture medium of the invention was added to the plate and the resultant was mixed well. After surface disinfection, the plate was placed in a 37°C, 5% CO₂ incubator (purchased from Thermo Fisher) for culture.

Human primary acute myeloid leukemia cells before culture and after culture for 7 days were transferred into 15 mL centrifuge tubes, respectively and centrifuged at 1500 rpm for 5 minutes at room temperature. The supernatant was discarded, the cell pellet was diluted with 2 mL of 1x PBS, and evenly divided into 2 parts (one part was used for the experimental group to be double marked by leukocyte marker(APC Mouse Anti-Human CD45 (purchased from BD, 560973)) and myeloid marker (BB515 Mouse Anti-Human CD33 (purchased from BD, 564588)) ; the other part was used as a control group) in 1.5 mL centrifuge tubes, which are then centrifuged at room temperature for 5 minutes at 1500 rpm. The supernatant was discarded, and 40 µL of 0.5% BSA (prepared in 1X PBS) was added into the centrifuge tube. Then the antibodies described above were added at 1:40 to the cells in the dark, and mixed well. No antibodies were added to the control group. The cells were incubated on ice for 1 to 2 hours. After the incubation, each tube was added with 1 mL 1 X PBS for resuspension and washing, and centrifuged at room temperature at 1500 rpm for 5 minutes. The supernatant was discarded, 300 µL of 1 X PBS was added to resuspend the cell pellet; flow cytometry (Beckman EVOS M500) was used to analyze the expression of the myeloid marker in the human primary acute myeloid leukemia cells before culture and after culture for 7 days, respectively.

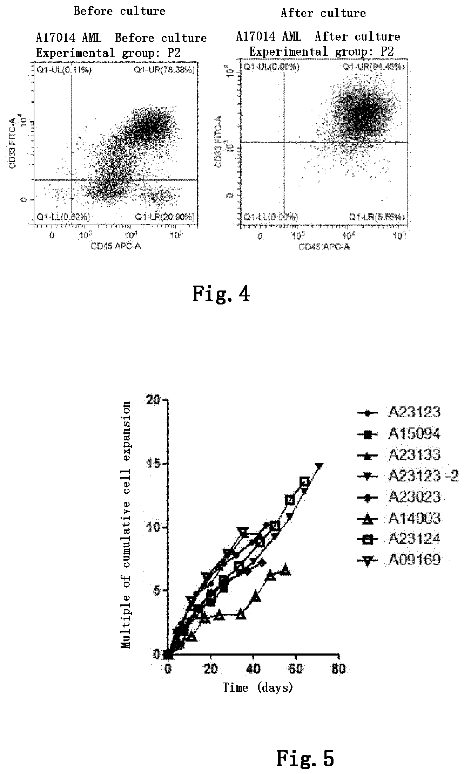

Figure 4 is the result of flow cytometric identification of human primary acute myeloid leukemia cells cultured using the culture medium for human primary acute myeloid leukemia cells of the invention. It can be confirmed from Figure 4 that after 7 days of continuous culture of human primary acute myeloid leukemia cells cultured in the medium of the invention, the proportion of myeloid leukemia cells increased by 16%.

### Example 5. Statistics of first culture period and cell number of human primary acute myeloid leukemia cells and calculation of Population Doubling (PD) value

According to the process of step (2)-3 of Example 1, human primary acute myeloid leukemia cells were obtained from 8 cases of human primary acute myeloid leukemia cell bone marrow samples (numbered A23123, A15094, A23133, A23123-2, A23023, A14003, A23124, A09169). The obtained human primary acute myeloid leukemia cells were inoculated into a 12-well plate at a viable cell density of 1 × 10⁶ cells/well and cultured using the medium of the invention. After 5 to 9 days of cell culture, the cells were passaged and counted, and the number of days of culture until the time of passage was regarded as a culture cycle. Under this experimental condition, the expanded cells were expanded in different passages. After each passage, the cells counted and the corresponding culture cycle was recorded. PD value was calculated according to the formula, Population Doubling (PD) = 3.32*log10 (total number of cells after digestion / initial number of inoculated cells). For the formula, see Chapman et al., Stem Cell Research & Therapy 2014, 5: 60.

Figure 5 shows the growth curves of 8 cases of primary cells cultured using the culture medium for human primary acute myeloid leukemia cells of the invention drawn by Graphpad Prism software. The abscissa represents the days of cell culture, and the ordinate represents the multiple of cumulative cell proliferation, i.e., the multiple of cell expansion in the culture cycle. The larger the value, the more times the cells are expanded within a certain cycle, that is, the more cells are expanded. The slope represents the rate of cell expansion. It can be confirmed from Figure 5 that when the human primary acute myeloid leukemia cells cultured in the culture medium of the invention were continuously cultured and expanded for at least 45 days, the cell expansion rate remained basically unchanged and the cells still had the ability to continue to expand.

### Example 6. Comparison of culture effects with the prior art culture medium

### (1) Preparation of Control Culture Medium

Control culture medium of the prior art (Silvia Ravera et al., Scientific Reports, (2020) 10:16519) was prepared with the formulation of 1640 culture medium (purchased from Corning, 10-040-CVR) + 10 ng/mL IL-15 (purchased from Sino Biological) + 10 ng/mL IL-4 (purchased from Sino Biological) + 10% FBS (purchased from excelllbio, FND500) (hereinafter referred to as "Control Culture Medium").

### (2) Obtaining and culturing of primary human acute myeloid leukemia cells

Human primary acute myeloid leukemia cells were obtained from a human primary acute myeloid leukemia bone marrow sample (A10093) according to the process of step (2)-3 of Example 1, inoculated in a 12-well plate at a viable cell density of 1 × 10⁶ cells/well, and cultured in the culture medium of the invention and Control Culture Medium, respectively.

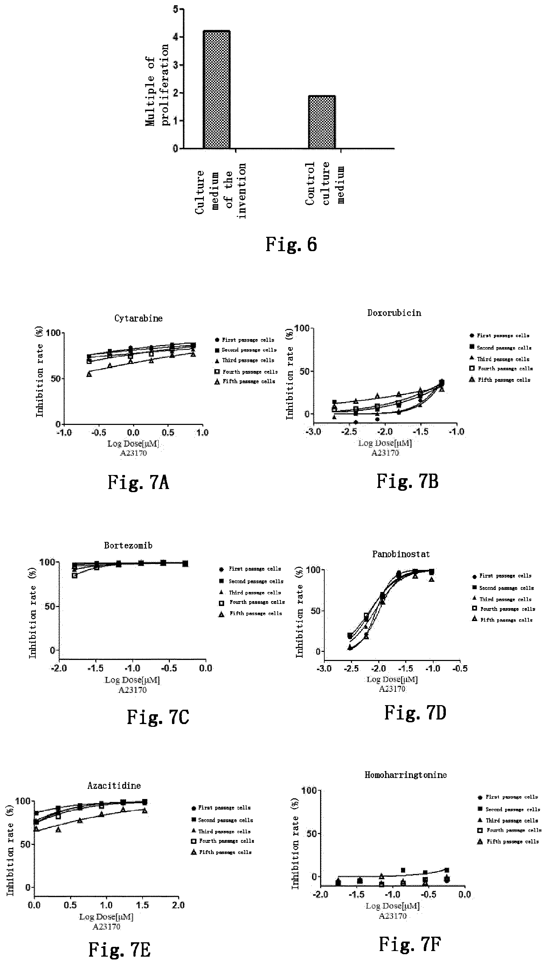

On the 7th day of culture, the 12-well plate was taken out. The cell culture was transferred into a 15 mL centrifuge tube, centrifuged at 1500 rpm for 5 minutes at room temperature, and the cell pellet was resuspended in 1 mL of culture medium. 12 µL of the resuspended cell suspension was mixed well with 12 µL of trypan blue dye (Sangon Biotech (Shanghai) Co., Ltd.), and 20 µL of the mixture was added into a cell counting plate (Countstar, specifications: 50 pieces/box). The total number of cells was counted with a cell counter (Countstar, IC1000). The counting results are shown in Figure 6.

According to the results in Figure 6, it can be seen that compared with the Control Culture Medium, the culture medium for human primary acute myeloid leukemia of the invention can significantly promote the expansion of human primary acute myeloid leukemia cells, and the effect is better than that of the Control Culture Medium.

### Example 7. Use of human primary acute myeloid leukemia cells expanded using the culture medium of the invention for drug screening and efficacy evaluation

### 1. Culture and plating of cells

According to the same process as in Example 1, human primary acute myeloid leukemia cells (numbered A23170) were isolated and used as a generation, and cultured using the culture medium for human primary acute myeloid leukemia cells of the invention until the cells were expanded to 85 %, which were then passaged. The cells were passaged and counted according to the steps in Example 1. The cells were placed in a loading slot (purchased from Corning) at a viable cell density of 1 × 10⁵ cells/mL and mixed thoroughly. Then, they were placed in a 384-well opaque white cell culture plate (purchased from Corning), with a volume of 50 µL per well and a cell number of 5,000 cells/well. The plate was sealed by adding the culture medium for human primary acute myeloid leukemia cells of the invention from the edge of the plate, and the sample names, dosing times and testing times of CellTiter-Glo (Promega) were marked on the plate. The surface of the plate was disinfected with 75% alcohol (LIRCON), and the plate was cultured in a 37°C, 5% CO₂ incubator, and dosed 24 hours later. Cells of the 1st, 2nd, 3rd, 4th and 5th passages of culture were respectively obtained for drug screening, and the drug sensitivity of the primary cells cultured using the culture medium of the invention for continuous passage was tested.

### 2. Preparation of candidate drugs

Six drugs (Cytarabine, Doxorubicin, Bortezomib, Panobinostat, Azacitidine, and Homoharringtonine; all purchased from MCE) in 6 concentration gradients were prepared according to the following table, which were added to a 384-well plate (Thermo Fisher) in a volume of 30 µL per well and stored for use.

**Table 3 Settings of drug concentration**

| Number | Drug names | Concentration gradient (µM) | | | | | |
|---|---|---|---|---|---|---|---|
| 1 | Cytarabine | 7.26 | 3.63 | 1.815 | 0.9075 | 0.45375 | 0.226875 |
| 2 | Doxorubicin | 0.062 | 0.031 | 0.0155 | 0.00775 | 0.003875 | 0.0019375 |
| 3 | Bortezomib | 0.52 | 0.26 | 0.13 | 0.065 | 0.0325 | 0.01625 |
| 4 | Panobinostat | 0.094 | 0.047 | 0.0235 | 0.01175 | 0.005875 | 0.0029375 |
| 5 | Azacitidine | 34 | 17 | 8.5 | 4.25 | 2.125 | 1.0625 |
| 6 | Homoharringtonine | 0.56 | 0.28 | 0.14 | 0.07 | 0.035 | 0.0175 |

### 3. High-throughput dosing

The prepared drug plate was taken out and kept at room temperature. The plate was centrifuged at room temperature for 1 minute at 1,000 rpm in a centrifuge (Beckman), and then taken out. A high-throughput automated workstation (JANUS, Perkin Elmer) was used for high-throughput dosing. To each well of the 384-well plate with cultured human primary acute myeloid leukemia cells was added 0.1 µL of the candidate drugs of corresponding concentrations. After dosing, the surface of 384-well plate was disinfected and moved to a incubator. The cell viability was measured after 72 hours.

### 4. Measurement of cell viability

CellTiter-Glo luminescent reagent (Promega) was taken out from a 4°C refrigerator, and 10 mL of the reagent was added into the loading slot. The 384-well plate to be tested was taken out from the incubator, and 10 µL CellTiter-Glo luminescent reagent was added into each well. After being standed for 10 minutes, the test was conducted by using a multifunctional microplate reader (Envision, Perkin Elmer).

### 5. Data processing

According to the formula, cell inhibition rate (%) = 100% - chemiluminescence value of the drug-dosed well / chemiluminescence value of the control well * 100%, the cell inhibition rate of cells treated with different drugs was calculated, and the half-inhibition rate (IC₅₀) of drugs on cells was calculated by the graphpad prism software. The results are shown in Figures 7A-7F.

It can be confirmed from Figures 7A-7F that when the human primary acute myeloid leukemia cells cultured from the culture medium for human primary acute myeloid leukemia cells of the invention were used for drug screening, the inhibitory effects of the same drug on the cultured cells of different passages remain substantially the same (the inhibition curve are substantially consistent). The cells from the same patient have different sensitivities to different drugs at the maximum blood concentration in the human body. According to the results, the effectiveness of the drug in clinical use in patients with human primary acute myeloid leukemia can be judged. At the same time, the results indicate that the sensitivity of tumor cells of different passages obtained according to the culture method of the invention to the drugs is stable.

### Industrial Applicability

The invention provides a primary cell culture medium and a culture method for culturing human primary acute myeloid leukemia cells *in vitro,* and the cultured cells can be used for efficacy evaluation and screening of drugs. Therefore, the invention is suitable for industrial applications.

Although the invention has been described in detail herein, the invention is not limited thereto. Those skilled in the art can make modifications according to the principles of the invention. Therefore, any modifications made according to the principles of the invention should be understood to fall within protection scope of the invention.

## Claims

1. A culture medium for human primary acute myeloid leukemia cells, **characterized in that**, comprising
a glutamine additive, non-essential amino acid(s), human interleukin-6, human interleukin-7, human interleukin-3, recombinant human FLT3 Ligand, recombinant human macrophage colony-stimulating factor, and human stem cell factor.

2. The culture medium for human primary acute myeloid leukemia cells of claim 1, **characterized in that**, the culture medium for human primary acute myeloid leukemia cells satisfies any one or more or all of the following conditions:
(1) the amount of the glutamine additive in the culture medium is 0.5 mM to 4 mM;
(2) the non-essential amino acid(s) is/are one or more selected from the group consisting of glycine, alanine, asparagine, aspartic acid, glutamic acid, proline and serine, and the amount of the non-essential amino acid(s) in the culture medium is 12.5 µM to 200 µM;
(3) the amount of human interleukin-6 in the culture medium is 1.89 ng/mL to 17 ng/mL;
(4) the amount of human interleukin-7 in the culture medium is 1.89 ng/mL to 51 ng/mL;
(5) the amount of human interleukin-3 in the culture medium is 1.89 ng/mL to 153 ng/mL;
(6) the amount of the recombinant human FLT3 Ligand in the culture medium is 3 ng/mL to 81 ng/mL;
(7) the amount of the recombinant human macrophage colony-stimulating factor in the culture medium is 1 ng/mL to 81 ng/mL;
(8) the amount of the human stem cell factor in the culture medium is 1 ng/mL to 81 ng/mL.

3. The culture medium for human primary acute myeloid leukemia cells of claim 1 or 2, **characterized in that**, further comprising a Basal Medium which comprises an initial medium selected from the group consisting of monocyte serum-free medium and RPMI-1640, fetal bovine serum, and one or more antibiotic(s) selected from the group consisting of streptomycin/penicillin, amphotericin B, and Primocin.

4. A method for culturing human primary acute myeloid leukemia cells, **characterized in that**,
the human primary acute myeloid leukemia cells are cultured using the culture medium for human primary acute myeloid leukemia cells according to any one of claims 1 to 3.

5. A method for screening or efficacy evaluating of drugs for human primary acute myeloid leukemia, **characterized in that**, comprising the steps of
(1) culturing the human primary acute myeloid leukemia cells by the method for culturing human primary acute myeloid leukemia cells of claim 4;
(2) selecting the drug to be tested and diluting the drug into different concentration gradients;
(3) adding the drug which has been diluted to the cells cultured and obtained in step (1), and detecting the cell viability.
